# FASCICULE DE BREVET EUROPEEN

(11) **EP 4 410 345 B1**
(45) Date de publication et mention de la délivrance du brevet: **11.03.2026**
(21) Numéro de dépôt: 24152952.8
(22) Date de dépôt: 19.01.2024
(51) Int. Cl.: A61M 16/12, F16B 2/10, F16B 31/02

(54) **APPAREIL MÉDICAL DE FOURNITURE DE GAZ, TEL DU NO, ÉQUIPÉ D'UN DISPOSITIF DE FIXATION À UN SUPPORT ALLONGÉ**
MEDIZINISCHES GERÄT ZUR GASVERSORGUNG, WIE NO, MIT EINER VORRICHTUNG ZUR BEFESTIGUNG AN EINEM LÄNGLICHEN TRÄGER
MEDICAL GAS SUPPLY APPARATUS, SUCH AS NO, WITH A DEVICE FOR ATTACHMENT TO AN ELONGATE SUPPORT

(30) Priorité: 03.02.2023 FR 2301039
(43) Date de publication de la demande: 07.08.2024
(73) Titulaire: INOSYSTEMS, 92160 Antony (FR)
(72) Inventeur: TIRILLY, Nicolas, 92160 Antony (FR); KAHALE, Christian, 92160 Antony (FR)
(74) Mandataire: Air Liquide

(56) Documents cités:
- EP-A1- 2 937 056
- WO-A1-2005/033524
- WO-A1-2021/178829
- US-A1- 2010 302 655
- US-A1- 2011 147 550
- US-A1- 2019 192 763

## Description

L'invention concerne un appareil médical de fourniture de gaz, tel du NO, typiquement de mélange gazeux NO/N₂, équipé d'un dispositif de fixation permettant de fixer, c'est-à-dire d'accrocher et maintenir, l'appareil à un support allongé, c'est-à-dire longiforme, en particulier de type tringle ou barreau.

Pendant leur utilisation, certains appareils médicaux doivent être fixés, c'est-à-dire accrochés et maintenus en position, sur des supports ayant des structures différentes, typiquement des supports de type tringle ou barreau ayant des sections carrées, rectangulaires ou cylindriques.

Ainsi, certains appareils médicaux de fourniture gaz, tel ceux de fourniture de NO, i.e. typiquement mélange NO/N₂, doivent pouvoir être transportés dans des véhicules d'urgence, tel qu'une ambulance, un avion ou autre. Pendant le transport, pour éviter leur chute, ils doivent être solidement fixés à un support agencé dans le véhicule (e.g. tringle rectangulaire ou carrée) ou sur le brancard (e.g. barreau cylindrique) sur lequel se trouve le patient auquel du gaz, e.g. du NO, est administré au moyen de ces appareils. Un exemple d'appareil de délivrance de NO est décrit par EP-A-3906955.

A cette fin, on peut agencer un dispositif de fixation dédié, par exemple un dispositif à pince, sur la paroi arrière du boîtier externe de l'appareil médical considéré, c'est-à-dire sur sa carcasse ou coque périphérique. Ainsi, EP-A-3639975 enseigne une pince de serrage permettant de fixer un appareil sur des supports de tailles et formes différentes, notamment un appareil médical en milieu hospitalier. Elle est formée d'une partie basse comprenant une mâchoire inférieure concave et un prolongement arrière formant une première poignée de serrage, et d'une partie supérieure comprenant une mâchoire supérieure concave et une portion arrière servant de deuxième poignée. Ces parties portent plusieurs axes de pivotement permettant aux deux parties de pivoter. Deux axes servent aussi de points d'ancrage pour un bras de verrouillage servant à ajuster l'écartement des mâchoires. Une molette de réglage rotative agencée autour du bras de verrouillage permet de régler sa longueur, donc l'écartement des mâchoires. Une telle structure de pince est cependant complexe et peu aisée à manipuler, notamment la présence de la molette de réglage rotative complique son architecture.

Un autre dispositif à pince de serrage, conforme au préambule de la revendication 1, est décrit par WO2021/178829.

Un problème est dès lors de proposer un appareil médical de fourniture de gaz, en particulier de NO, équipé d'un dispositif de fixation permettant de fixer l'appareil médical à un support allongé, i.e. longiforme, de type tringle ou barreau, en évitant ou limitant tout ou partie des problèmes, risques et/ou inconvénients susmentionnés, que le support soit d'axe horizontal, vertical ou oblique.

Une solution de l'invention concerne un appareil médical de fourniture de gaz, en particulier de NO gazeux, tel un appareil de fourniture de mélange gazeux NO/N₂, comprenant une carcasse comprenant une paroi externe et un dispositif de fixation à un support allongé, agencé sur la paroi externe de ladite carcasse, dans lequel le dispositif de fixation comprend :
- un corps de pince et un élément de pince mobile en pivotement l'un par rapport à l'autre,
- au moins une partie du corps de pince est configurée en une première mâchoire et au moins une partie de l'élément de pince mobile étant configurée en une seconde mâchoire, lesdites première et seconde mâchoires étant agencées face à face pour former une pince de serrage, et
- un organe de manipulation rotatif, actionnable en rotation par un utilisateur, coopérant avec l'élément de pince, lorsqu'il est actionné, pour déplacer l'élément de pince respectivement au corps de pince et rapprocher ou éloigner la première mâchoire de la seconde mâchoire.

De plus :
- l'organe de manipulation rotatif comprend une tête destinée à être saisie manuellement par l'utilisateur et une tige d'actionnement comprend une portion filetée, et
- une pièce d'actionnement taraudée coopère avec la portion filetée de la tige d'actionnement et avec l'élément de pince pour opérer le pivotement de l'élément de pince par rapport au corps de pince de manière à rapprocher ou éloigner lesdites première et seconde mâchoires l'une de l'autre.

Selon l'invention, l'organe rotatif comprend en outre un système limiteur de couple agencé dans la tête de l'organe de manipulation rotatif, ledit système limiteur de couple comprenant un empilement de rondelles-ressorts coopérant avec une rondelle de pression.

Selon le mode de réalisation considéré, l'appareil médical de l'invention peut comprendre l'une ou plusieurs des caractéristiques suivantes :
- le corps de pince est fixe.
- le corps de pince est fixé à une platine-support.
- le corps de pince et l'élément de pince sont portés par une platine-support.
- la platine-support est fixée à la paroi externe de ladite carcasse, de préférence fixée par vissage.
- la platine-support comprend une plaque perforée.
- la platine-support est fixée à la face arrière de la carcasse de l'appareil.
- la portion filetée de la tige d'actionnement est agencée à une extrémité libre de la tige d'actionnement de l'organe de manipulation rotatif.
- la portion filetée de la tige d'actionnement comprend un filetage périphérique, c'est-à-dire portée par une partie de la surface périphérique de la tige d'actionnement.
- la tige d'actionnement a une forme cylindrique.
- la tige d'actionnement est en métal, tel de l'acier inoxydable.
- la portion filetée de la tige d'actionnement de l'organe de manipulation rotatif est configurée pour se déplacer dans le taraudage de la pièce d'actionnement taraudée lorsque l'utilisateur applique un mouvement de vissage horaire ou antihoraire à la tête de l'organe de manipulation rotatif.
- la pièce d'actionnement taraudée comprend un corps de pièce comprenant un alésage, i.e. un orifice traversant ou borgne ou analogue, dont la paroi interne est taraudée, c'est-à-dire porte un filetage.
- la pièce d'actionnement taraudée comprend un taraude, i.e. filetage interne, complémentaire du filetage de la portion filetée de la tige d'actionnement de l'organe de manipulation rotatif.
- la pièce d'actionnement taraudée est configurée être déplacée en translation, lorsque la tige d'actionnement de l'organe de manipulation rotatif vient agir sur la pièce d'actionnement taraudée.
- la pièce d'actionnement taraudée est configurée pour engendrer un déplacement angulaire, c'est-à-dire un pivotement, de l'élément de pince par rapport au corps de pince, et un déplacement en rapprochement ou en éloignement de la seconde mâchoire par rapport à la première mâchoire, selon le sens d'actionnement de la tige d'actionnement par l'utilisateur, lorsque la tige d'actionnement de l'organe de manipulation rotatif vient agir sur la pièce d'actionnement taraudée.
- la pièce d'actionnement taraudée est en métal, par exemple en acier inoxydable.
- la pièce d'actionnement taraudée constitue une pièce intermédiaire faisant la liaison entre la tige d'actionnement de l'organe de manipulation et l'élément de pince pivotant.
- la pièce d'actionnement taraudée constitue une pièce intermédiaire (i.e. de liaison) permettant de transformer le mouvement rotatif appliqué par l'utilisateur à l'organe de manipulation et à la tige d'actionnement en un mouvement translatif de sorte que ladite pièce d'actionnement puisse venir coopérer avec l'élément de pince pivotant pour le faire pivoter selon l'axe XX.
- le pivotement se fait selon un axe de pivotement (XX).
- la première zone de serrage de la première mâchoire présente un profil semi-circulaire, c'est-à-dire qu'elle a une forme de semi-cylindrique ou analogue, et la seconde zone de serrage de la seconde mâchoire présente un profil linéaire, c'est-à-dire qu'elle est de forme plate ou plane, ou inversement.
- la première mâchoire et la seconde mâchoire sont conformées pour permettre une prise en étau d'un support de section polygonale ou de section circulaire, en particulier une tringle de section carrée ou rectangulaire, ou un barreau de section circulaire, et en assurer un maintien ferme, i.e. solide.
- la tête de l'organe de manipulation rotatif est creuse, c'est-à-dire qu'elle comprend une chambre interne.
- la tête de l'organe de manipulation est en polymère ou en métal, par exemple en aluminium ou un alliage d'aluminium.
- le système limiteur de couple est agencé dans une chambre ou un volume interne de la tête de l'organe de manipulation.
- le système limiteur de couple comprend un empilement de plusieurs rondelles-ressorts comprenant moins de 12 rondelles-ressorts, de préférence de 2 à 8 rondelles-ressorts.
- un moyen de maintien, telle une vis ou analogue, permet se solidariser l'empilement de rondelles-ressorts à la tige d'actionnement.
- l'empilement de rondelles-ressorts est maintenu entre une tête du moyen de maintien, telle une tête de vis ou analogue, et une rondelle de pression, aussi appelée rondelle d'arrêt.
- l'empilement de rondelles-ressorts vient appuyer sur la rondelle de pression agencée dans la tête de l'organe de manipulation rotatif, de préférence y exerce une force de poussée (FdP) axiale (axe AA).
- les rondelles-ressorts sont élastiques.
- la tête de l'organe de manipulation rotatif comprend un épaulement annulaire interne agencé, i.e. pris en sandwich, entre la rondelle de pression et un élément de socle solidaire de la tige d'actionnement.
- l'épaulement annulaire est traversé par au moins un logement à goupille comprenant une pièce-mobile mobile en translation au sein dudit logement à goupille.
- la pièce-mobile est une goupille ou analogue.
- l'épaulement annulaire est traversé par plusieurs logements à goupille comprenant chacun une pièce-mobile, par exemple 2 ou 3 logements à goupille, ou plus.
- les logements à goupille sont agencés et/ou angulairement répartis sur l'épaulement annulaire.
- le (ou les) logements à goupille est un perçage, de préférence axial AA, traversant l'épaulement annulaire.
- l'épaulement annulaire a une forme de couronne.
- l'élément de socle comprend au moins un logement-borgne aménagé sur une surface annulaire supérieure de l'élément de socle située en regard de l'épaulement.
- l'élément de socle comprend plusieurs logements-borgnes aménagés sur la surface annulaire supérieure de l'élément de socle.
- le système limiteur de couple peut adopter au moins une position embrayée et une position débrayée, en fonction du couple de serrage maximal du système limiteur de couple, c'est-à-dire selon que le couple de serrage maximal est atteint ou non.
- la pièce-mobile est partiellement insérée dans ledit au moins un logement borgne de l'élément de socle lorsque le système limiteur de couple est dans ladite au moins une position embrayée.
- en position embrayée, la tête de l'organe de manipulation rotatif est couplée à l'élément de socle, via l'épaulement annulaire, de sorte qu'un mouvement de rotation, i.e. horaire ou antihoraire, appliqué par l'utilisateur à la tête de l'organe de manipulation rotatif, entraîne simultanément une rotation de la tête de l'organe de manipulation rotatif et de l'élément de socle étant donné qu'ils sont solidaires/couplés l'un à l'autre.
- la pièce-mobile est positionnée en dehors dudit au moins un logement borgne de l'élément de socle lorsque le système limiteur de couple est en position débrayée.
- en position débrayée, la tête de l'organe de manipulation rotatif est découplée de l'élément de socle, de sorte qu'un mouvement de rotation (i.e. horaire ou antihoraire) appliqué par l'utilisateur à la tête de l'organe de manipulation rotatif n'entraîne pas/plus l'élément de socle, c'est-à-dire que la tête de l'organe de manipulation rotatif tourne librement sans coopérer avec l'élément de socle.
- en position débrayée, ladite au moins une pièce-mobile repousse la rondelle de pression en direction de l'empilement de rondelles-ressorts de sorte d'éloigner la rondelle de pression de l'épaulement annulaire de la tête de l'organe de manipulation rotatif.
- l'appareil est un appareil de fourniture de NO gazeux, en particulier de mélange gazeux NO/N₂, destiné à être raccordé à un circuit patient d'un ventilateur médical fournissant un gaz respiratoire, en particulier un mélange gazeux N₂/O₂ ou de l'air.
- l'appareil comprend en outre, agencés dans la carcasse, un circuit de gaz interne pour acheminer le gaz, des moyens de contrôle de débit du gaz véhiculé par le circuit de gaz interne, et des moyens de pilotage pour piloter les moyens de contrôle du débit.
- le circuit de gaz interne comprend un (ou des) passage de gaz.
- les moyens de contrôle de débit comprennent une ou des vannes pilotées, en particulier une ou des électrovannes.
- les moyens de pilotage comprennent au moins un microprocesseur.
- le (ou les) microprocesseur est agencé sur au moins une carte électronique.
- le (ou les) microprocesseur met en œuvre au moins un algorithme.
- il comprend des premiers moyens de raccordement à une ou des sources de gaz, telles des bouteilles de gaz, i.e. de mélange NO/N₂ et/ou d'oxygène.
- il comprend des deuxièmes moyens de raccordement au circuit patient du ventilateur médical.
- il comprend des troisièmes moyens de raccordement à un capteur de débit agencé sur ou dans le circuit patient du ventilateur médical.
- il comprend un écran d'affichage, de préférence porté par la face avant ou façade de la carcasse de l'appareil.
- il comprend des moyens d'alimentation électrique, telle qu'un ou des câbles de raccordement au secteur (110/220V) et/ou une prise électrique.
- alternativement, l'appareil est un ventilateur médical de fourniture d'air ou de mélange air/oxygène.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un dispositif de fixation pour appareil médical selon la présente invention, en particulier pour appareil de fourniture de NO,
Fig. 2 schématise la forme des mâchoires (vue de côté) du dispositif de fixation de Fig. 1,
Fig. 3 schématise (vue de face) un mode de réalisation d'un appareil médical de fourniture de NO équipé du dispositif de serrage selon la présente invention,
Fig. 4 est une vue de côté de l'appareil de Fig. 3 montrant le dispositif de fixation de Fig. 1,
Fig. 5 schématise (vue en coupe) un mode de réalisation de l'organe rotatif intégrant un limiteur de couple du dispositif de serrage selon la présente invention de Fig. 1,
Fig. 6A est une vue grossie d'une partie de l'organe rotatif intégrant le limiteur de couple du dispositif de serrage de Fig. 5,
Fig. 6B est une vue en coupe selon le plan B-B de Fig. 6A, et
Fig. 7 à Fig. 9 schématisent le fonctionnement du limiteur de couple du dispositif de serrage de Fig. 5 et Fig. 6A.

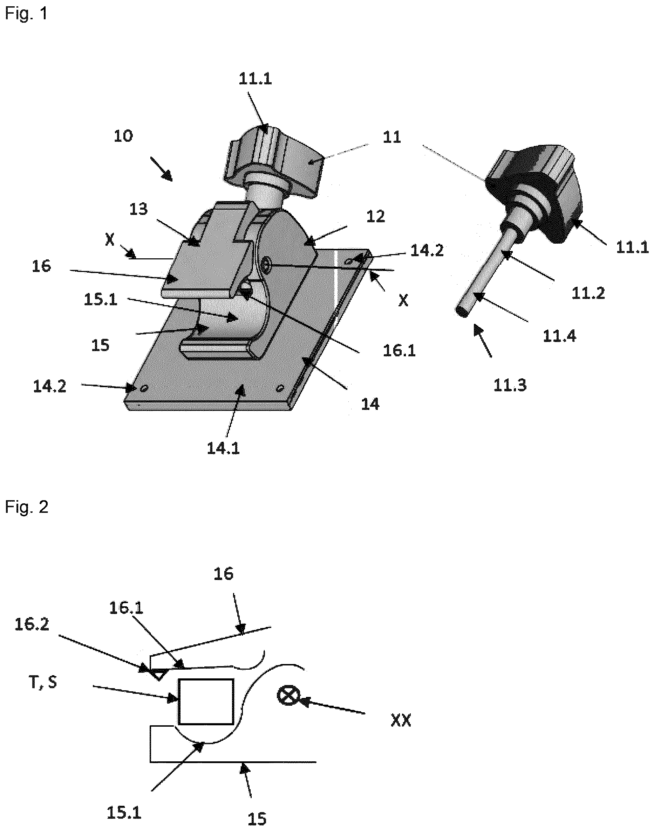

Fig. 1 schématise un mode de réalisation d'un dispositif de fixation 10 pour appareil médical 1 selon la présente invention, en particulier pour appareil de fourniture de NO.

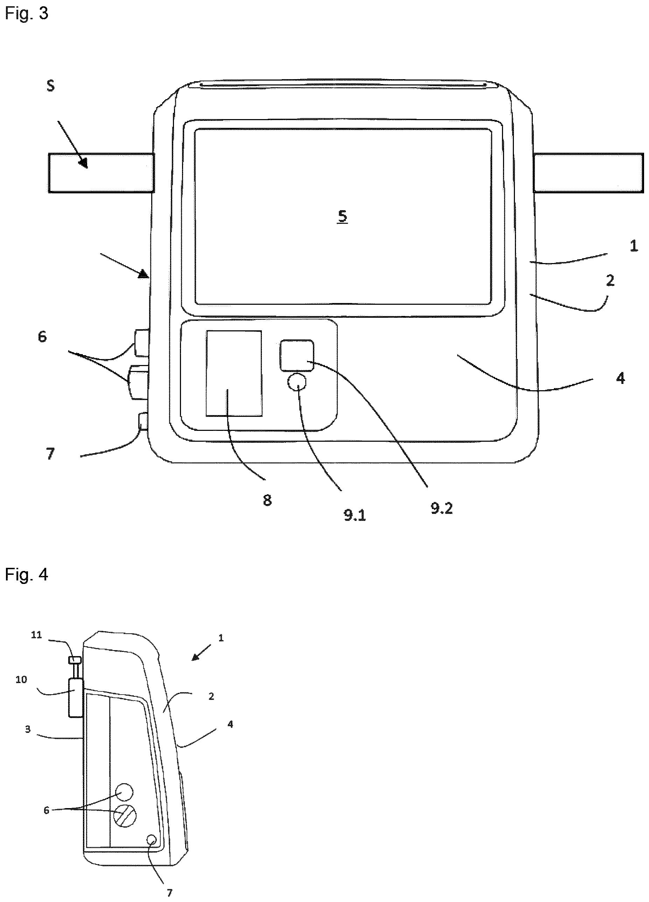

Le dispositif de fixation 10 selon l'invention de Fig. 1 est destiné à équiper un appareil médical 1, tel que schématisé sur Fig. 3 et Fig. 4, en particulier un appareil de fourniture de NO, i.e. de mélange NO/azote, par exemple du type de celui décrit par EP-A-3906955 ou de celui commercialisé par Air Liquide Healthcare^{®} sous la dénomination commerciale SoKinox^{®}.

Il est conçu pour permettre d'accrocher et fixer l'appareil médical 1 à un support S allongé, c'est-à-dire longiforme ou longiligne, telle une tringle T de section externe (i.e. périmètre) rectangulaire ou carrée, ou un barreau de forme cylindrique, c'est-à-dire de section externe (i.e. périmètre) circulaire, en particulier une tringle horizontale ou un barreau horizontal ou vertical, comme illustré en Fig. 3.

Fig. 3 et Fig. 4 schématisent des vues des faces avant 4 (façade) et arrière 3 (dos) d'un mode de réalisation d'un appareil médical 1 de fourniture de NO équipé du dispositif de serrage 10 selon la présente invention, lequel appareil médical 1 est montré accroché à un support allongé S en Fig. 3.

Le dispositif de fixation 10 est porté par la face arrière 3 du boitier externe 2, c'est-à-dire de la carcasse ou coque périphérique rigide, de l'appareil médical 1 comme schématisé en Fig. 4, par exemple un boitier 2 rigide en polymère.

La face avant 4 de l'appareil médical 1 de fourniture de NO comprend un écran d'affichage 5 d'informations, avantageusement un écran à dalle tactile, de préférence à affichage en couleurs, permettant d'afficher des informations, des données, des graphiques, des mesures ou toute autre donnée utile à l'utilisateur, typiquement un personnel médical, tel un médecin. L'écran d'affichage 5 d'informations à dalle tactile permet également à l'utilisateur d'opérer des choix, des réglages, des sélections, des validations ou autres via une ou des touches tactiles et/ou des menus de sélection ou analogues s'affichant sur l'écran 5.

L'appareil médical 1 de fourniture de NO comprend par ailleurs un ou des boutons de réglage 6 ou similaires, par exemple agencés sur l'une des faces latérales de l'appareil 1, ainsi qu'un ou des connecteurs ou raccords 7 de raccordement de tuyaux flexibles véhiculant du gaz, tel un mélange NO/N2, de l'oxygène ou leurs mélanges.

De préférence, il comprend aussi une entrée d'une ligne d'échantillonnage de gaz 8 avec un dispositif de type piège à eau, une entrée de mesure de débit 9.2 et une sortie d'injection 9.1 pour fournir le mélange NO/N₂.

Le dispositif de fixation 10 agencé sur la face arrière 3 du boitier 2 de l'appareil médical 1, comprend un organe de manipulation rotatif 11, un corps de pince 12 fixe et un élément de pince 13 mobile portés par une platine-support 14. Leur fonctionnement est détaillé ci-après.

Dans le mode de réalisation de Fig. 1, la platine-support 14 comprend une (ou des) plaque 14.1 formant embase, par exemple métallique, laquelle est percée de plusieurs trous 14.2 de passage de vis ou analogue servant à la fixation par vissage de la platine-support 14 à la face arrière 3 de la carcasse 2 de l'appareil médical 1, en particulier un appareil de fourniture de NO.

Dans ce cas, une partie du corps de pince 12 est configurée pour former une première mâchoire 15 de pince et une partie de l'élément de pince 13 mobile est configuré pour former une seconde mâchoire 16 de pince.

Les première et seconde mâchoires 15, 16 sont situées en regard l'une de l'autre, c'est-à-dire agencées face à face, pour former une pince de serrage permettant de recevoir des supports S allongés, c'est-à-dire longiforme, de différentes formes, à savoir indifféremment un support de section polygonale, e.g. carrée ou rectangulaire, ou de section circulaire, typiquement une tringle T portée par une paroi de véhicule ou d'un bâtiment, ou un barreau cylindrique de brancard, de lit d'hôpital ou analogue. Grâce à un serrage par rapprochement des première et seconde mâchoires 15, 16, le support S allongé, qu'il soit de section polygonale ou circulaire, peut être maintenu efficacement entre lesdites mâchoires 15, 16, en étant pris en sandwich ou en étau entre celles-ci 15, 16.

Afin d'améliorer la prise en étau du support allongé S et son maintien ferme entre elles, les première et seconde mâchoires 15, 16 sont conformées pour présenter des zones de serrage 15.1, 16.1 de formes particulières.

Ainsi, dans le mode de réalisation proposé en Fig. 1, la première zone de serrage 15.1 de la première mâchoire 15 présente un profil ou section semi-circulaire, c'est-à-dire qu'elle a une forme de semi-cylindrique ou analogue, alors que la seconde zone de serrage 16.1 de la seconde mâchoire 16 présente un profil rectiligne, i.e. droit (non courbé), c'est-à-dire qu'il présente une surface plate ou plane. Ces profils sont mieux visibles en Fig. 2.

Selon un mode de réalisation, la seconde zone de serrage 16.1, à savoir la surface plane visible en Fig. 2, est préférentiellement terminée par un rebord 16.2 permettant d'éviter ou limiter toute sortie intempestive par glissement du support allongé S, telle une tringle T polygonale ou un barreau cylindrique.

Bien que moins préféré, selon un autre mode de réalisation, les profils des première et seconde zones de serrage 15.1, 16.1 pourraient être inversés.

Dans tous les cas, ces profils particuliers de ces première et seconde zones de serrage 15.1, 16.1 permettent de pouvoir opérer un serrage efficace du support S allongé, quelle que soit sa forme, c'est-à-dire non seulement une tringle T de section polygonale, e.g. carrée ou rectangulaire, comme schématise en Fig. 2, mais aussi d'un barreau cylindrique, i.e. de section circulaire, ovale ou ellipsoïdal. En effet, le profil semi-circulaire de la première zone de serrage 15.1 de la première mâchoire 15 est bien adapté aux supports S de type barreau cylindrique, alors que le profil linéaire (i.e. plan) de la seconde zone de serrage 16.1 de la seconde mâchoire 16 est bien adapté aux supports S de type tringle T de section polygonale ou analogue. Dès lors, leur combinaison engendre un effet 'synergique' permettant de maintenir solidement les deux types de supports S.

Par ailleurs, conformément à la présente invention, l'organe de manipulation rotatif 11 qui coopère avec l'élément de pince 13 mobile, comprend une tête 11.1 et une tige d'actionnement 11.2, laquelle est filetée 11.4 à son extrémité libre 11.3.

La tête 11.1 de l'organe de manipulation rotatif 11 est destinée à être saisie manuellement par l'utilisateur et actionnée dans le sens horaire par exemple pour opérer un serrage par rapprochement des première et seconde zones de serrage 15.1, 16.1, l'une de l'autre, du fait d'un pivotement de la seconde mâchoire 16 en direction de la première mâchoire 15 fixe, ou inversement, un desserrage dans le sens antihoraire.

Dit autrement, l'actionnement de la tête 11.1 de l'organe de manipulation rotatif 11 permet de rapprocher ou éloigner la seconde mâchoire 16 de la première mâchoire 15.

Pour ce faire, la tige d'actionnement 11.2 comprenant une portion filetée 11.4 (i.e. avec filetage périphérique), de préférence située à une extrémité libre 11.3, qui va coopérer, lorsqu'elle est manipulée par l'utilisateur, avec une pièce d'actionnement taraudée (non visible), c'est-à-dire présentant un taraudage, qui est solidaire de l'élément de pince 13 mobile, i.e. pivotant, et qui agit sur celui-ci pour le faire pivoter.

La portion filetée 11.4 de la tige d'actionnement 11.2 coopère, lors d'un vissage ou dévissage, avec le taraudage de la pièce d'actionnement taraudée, étant donné que leurs filets sont configurés pour être complémentaires l'un de l'autre, i.e. mêmes filets.

Le taraudage de la pièce d'actionnement est un alésage, i.e. orifice ou analogue, aménagé au travers de la pièce d'actionnement dans la paroi périphérique duquel est aménagé un taraudage, i.e. filetage interne. La pièce d'actionnement taraudée est donc une pièce intermédiaire usinée, par exemple percée et filetée, de sorte de présenter un taraudage dans son perçage.

Le déplacement, de préférence en translation, de la pièce d'actionnement taraudée va engendrer un déplacement angulaire, c'est-à-dire un pivotement (selon axe XX), de l'élément de pince 13 par rapport au corps de pince 12, donc un déplacement en rapprochement ou en éloignement de la seconde mâchoire 16 par rapport à la première mâchoire 15 fixe, selon le sens d'actionnement de la tige d'actionnement 11.2, via sa tête 11.1, par l'utilisateur, c'est-à-dire un vissage ou dévissage de la tête 11.1 de l'organe de manipulation rotatif 11.

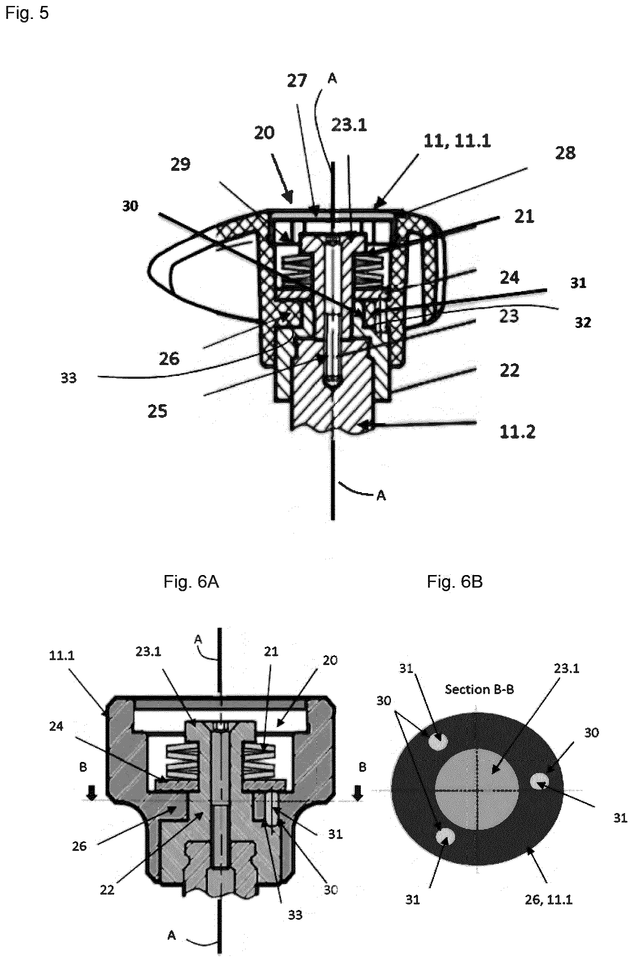

Avantageusement, comme schématisé en Fig. 5, Fig. 6A et Fig. 6B, l'organe rotatif 11, en particulier la tête 11.1 qui est (au moins en partie) creuse, intègre un système limiteur de couple 20 qui fonctionne par friction via un empilage de rondelles-ressorts 21, telles des rondelles dites 'Belleville' ou analogue.

Typiquement, un empilage ou empilement de 2 à 8 rondelles-ressorts 21. Ces rondelles-ressorts 21 sont positionnées sur un élément de socle 22 venant coiffer, c'est-à-dire agencé sur, l'extrémité supérieure de la tige d'actionnement 11.2. Une rondelle de pression 24, aussi appelée rondelle d'arrêt, est prise en sandwich entre l'empilement de rondelles-ressorts 21 et l'élément de socle 22. L'élément de socle 22 est couplé à la tige d'actionnement 11.2 et l'ensemble élément de socle 22/ tige d'actionnement 11.2 peut être entrainé en rotation au tour de l'axe AA, comme expliqué ci-après et illustré sur Fig. 5 et Fig. 6A, en particulier lors d'un vissage ou dévissage de la tête 11.1 de l'organe de manipulation rotatif 11 par l'utilisateur.

La tête 11.1 de l'organe de manipulation rotatif 11 comprend un épaulement annulaire 26 interne, à savoir une paroi interne, de préférence de forme annulaire, faisant saillie radialement (i.e. dirigé vers l'axe AA) dans la tête 11.1, comme illustré en Fig. 5 et Fig. 6A. Cet épaulement 26 interne est pris en sandwich entre la rondelle de pression 24 et l'élément de socle 22.

De préférence, cet épaulement 26 interne a une forme de couronne. L'épaulement 26 interne est traversé, de préférence axialement (axe AA), par au moins un logement à goupille 30, de préférence plusieurs logements à goupille 30 répartis sur l'épaulement 26, dans lequel (lesquels) est logée une pièce-mobile 31, en particulier une goupille ou analogue, c'est-à-dire qu'une pièce-mobile 31 est agencée dans chaque logement à goupille 30, comme illustré sur Fig. 5 et Fig. 6A.

Sur Fig. 6B qui est une vue en coupe selon B-B de Fig. 6A, on a représenté un mode de réalisation où sont présents plusieurs logements à goupille 30 comprenant chacun une pièce-mobile 31, lesquels sont agencés et angulairement répartis sur l'épaulement annulaire 26, c'est-à-dire en forme de couronne.

Le ou les logements à goupille 30 sont des perçages traversant axialement l'épaulement 26 interne en forme de couronne. La (ou les) pièce-mobile 31 est (sont) configurée pour se déplacer axialement, i.e. en translation, au sein dudit logement à goupille 30.

Le socle 22 comprend par ailleurs au moins un logement-borgne 32, de préférence plusieurs logements-borgnes 32, aménagé sur une surface annulaire supérieure 33 située en regard de l'épaulement 26, de sorte que la (ou les) pièce-mobile 31 puisse venir se loger dans au moins un logement-borgne 32, lorsque le système limiteur de couple 20 est en position embrayée et apte à entrainer l'élément de socle 22 en rotation en même temps que la tête 11.1 de l'organe de manipulation rotatif 11 comprenant l'épaulement 26. Autrement dit, lorsqu'une (ou des) pièce-mobile 31 est partiellement insérée dans un (des) logement-borgne 32, la tête 11.1 de l'organe de manipulation rotatif 11 et l'élément de socle 22 sont solidarisés l'un à l'autre et sont mobiles « d'un bloc » en rotation atour de l'axe AA, par exemple lorsque l'utilisateur exerce un mouvement de serrage dans le sens horaire de l'organe de manipulation rotatif 11 de manière à rapprocher les première et seconde mâchoires 15, 16 l'une de l'autre.

La rondelle de pression 24 vient appuyer sur la pièce-mobile 31 pour la repousser vers l'élément de socle 22 tant que le système limiteur de couple 20 ne se « déclenche pas » comme expliqué ci-après, en cas de serrage au-delà du couple maximal.

L'ensemble comprenant les rondelles-ressorts 21, la rondelle de pression 24 et le socle 22 coopèrent avec la tige d'actionnement 11.2 et y sont fixés par un moyen de maintien 23, telle une vis ou analogue, venant se solidariser à l'extrémité supérieure de la tige d'actionnement 11.2, par exemple se fixer par vissage dans un logement 25 aménagé dans l'extrémité supérieure de la tige d'actionnement 11.2.

Comme on le voit sur Fig. 5 et Fig. 6A, les rondelles-ressorts 21 sont maintenues entre la tête 23.1 du moyen de maintien 23, telle une vis, et la rondelle de pression 24. Plus précisément, le moyen de maintien 23, telle une vis ou analogue, traverse les rondelles-ressorts 21, la rondelle de pression 24 et le socle 22.

Afin de permettre le montage du système limiteur de couple 20 dans la tête 11.1 qui est creuse, c'est-à-dire comprend une chambre ou un volume interne 29, on prévoit un couvercle 27 amovible venant fermer une ouverture 28 aménagée sur la tête 11.1, laquelle ouverture 28 donne accès au volume interne 29 de la tête 11.1 de l'organe rotatif 11.

Afin, d'effectuer le serrage sur une barre ou un barreau, il faut tourner la tête 11.1 de l'organe rotatif 11 dans le sens des aiguilles d'une montre par exemple.

Comme schématisé en Fig. 5, Fig. 6A et Fig. 7, lors d'un serrage dans le sens de serrage (SdS), la tête 11.1 de l'organe rotatif 11 entraine le socle 22, donc aussi la tige d'actionnement 11.2, en rotation dans le sens de serrage (SdS), typiquement le sens horaire. Les rondelles-ressorts 21, i.e. rondelles élastiques calibrées, exercent une force de poussée (FdP), c'est-à-dire exercent un effort dirigé vers l'élément de socle 22, et repoussent alors la rondelle de pression 24 en direction de l'élément de socle, ce qui repousse simultanément la (ou les) pièce-mobile 31, telle une goupille ou analogue, dans le logement-borgne 32 porté par la surface annulaire supérieure 33 de l'élément de socle 22.

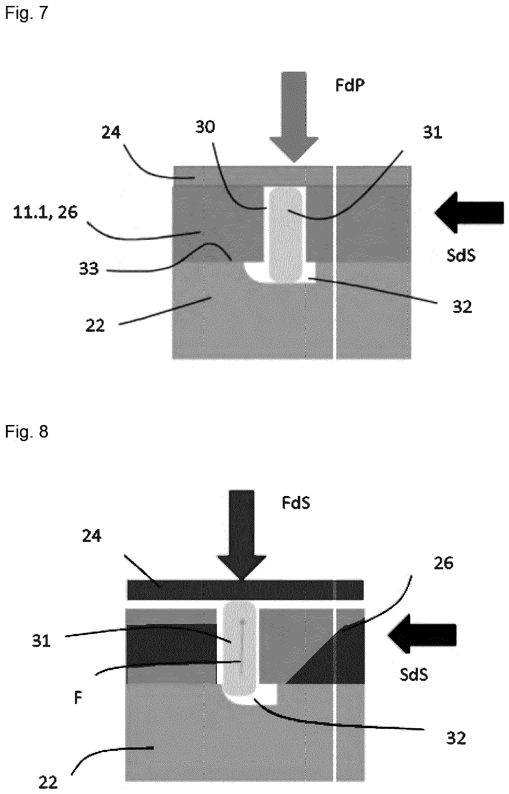

Tant que le couple de serrage est inférieur à une valeur maximale, la pièce-mobile 31 est ou reste partiellement insérée dans un (un des) logement-borgne 32, la tête 11.1 de l'organe de manipulation rotatif 11 et l'élément de socle 22 sont solidarisés l'un à l'autre et sont mobiles « d'un bloc » en rotation atour de l'axe AA, c'est-à-dire qu'ils sont en position embrayée, comme illustré en Fig. 7 et Fig. 8. En fait, le couple de serrage nominal est défini par les efforts de frottement engendrés par la déformation élastique des rondelles-ressorts 21 au fur et à mesure de leur serrage, c'est-à-dire de leur écrasement progressif entre la tête 23.1 du moyen de maintien 23, i.e. vis ou analogue, et la rondelle de pression 24.

On voit qu'en poursuivant le serrage comme illustré en Fig. 8, les forces de pression (FdP) appliquées par les rondelles-ressorts 21 tendent à diminuer du fait de la déformation progressive des rondelles-ressorts 21, ce qui engendre un déplacement axial de la pièce-mobile 31 dans le logement 30 (voir flèche F dirigée vers le haut sur Fig. 8), en éloignement par rapport l'élément de socle 22, engendrant alors sa sortie progressive du (ou des) logement-borgne 32 dans lequel elle est partiellement insérée. En effet, les efforts de pression appliqués par les rondelles-ressorts 21 deviennent alors insuffisants pour continuer à repousser la rondelle de pression 24 et cette dernière tend alors à s'éloigner de la surface supérieure de l'épaulement 26.

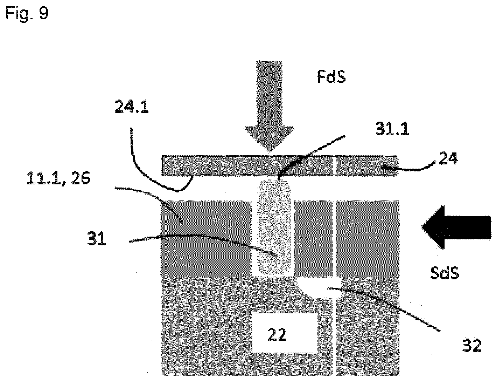

Lorsque le couple de serrage maximal est atteint, comme illustré en Fig. 9, le système limiteur de couple 20 de la tête 11.1 déclenche un "sur-engagement", c'est-à-dire qu'au-delà d'une certaine contrainte, les efforts de pression (FdP) des rondelles-ressorts 21 élastiques, donc sur la pièce 24, ne sont plus suffisant pour maintenir la fonction d'embrayage, i.e. la position embrayée, qui entraine l'ensemble du système en rotation. La rondelle de pression 24 faisant office de pièce d'entrainement tourne alors à vide car la (ou les) pièce-mobile 31, c'est-à-dire la (les) goupille ou analogue, n'est plus insérée dans le (les) logement-borgne 32 porté par la surface annulaire supérieure 33 de l'élément de socle 22, ce qui découple alors la tête 11.1 de l'organe rotatif 11 du socle 22, et permet alors à la tête 11.1 de l'organe rotatif 11 de tourner librement autour de l'axe AA sans entrainer l'élément de socle 22.

Autrement dit, lors du serrage, l'utilisateur ne pas dépasser le couple maximal car une fois le couple maximal atteint, la tête 11.1 de l'organe rotatif 11 va tourner à vide. Il devient alors impossible de serrer au-delà du couple maximal. La rondelle de pression 24 et l'épaulement 26 de la tête 11.1 de l'organe rotatif 11 sont alors débrayés l'un de l'autre, c'est-à-dire découplés.

Un tel agencement permet ainsi d'assurer un vissage optimal.

De préférence, un son audible par l'utilisateur, tel un bruit de « clic », créé par le débrayage du système permet de confirmer à l'utilisateur que le couple maximal a été atteint. Pour se faire, on peut prévoir des crans ou analogues sur la face inférieure 24.1 de la rondelle de pression 24 qui viendront alors frotter contre la tête 31.1 de la (ou de chaque) pièce-mobile 31 en mettant le son audible.

A l'inverse, un desserrage peut être effectué en tournant la tête 11.1 de l'organe rotatif 11 dans le sens contraire, par exemple le sens antihoraire. Les rondelles-ressorts 21 vont alors être progressivement moins comprimées et repousser progressivement la rondelle de pression 24 en direction de l'élément de socle 22. La (ou les) pièce-mobile 31, telle une goupille ou analogue, va alors pouvoir s'insérer à nouveau dans le (ou un des) logement-borgne 32 porté par la surface annulaire supérieure 33 de l'élément de socle 22, de sorte de coupler à nouveau le socle 22 et la tête 11.1 de l'organe rotatif 11, via l'épaulement 26, et ainsi permettre leur entrainement en rotation simultané pour dévisser l'organe de serrage rotatif 11 et ainsi éloigner les première et seconde mâchoires 15, 16 l'une de l'autre, c'est-à-dire opérer un desserrage.

D'une façon générale, selon l'invention, le système limiteur de couple 20 peut donc adopter au moins deux positions distinctes comprenant une position embrayée et une position débrayée, selon que le couple de serrage maximal est atteint ou non.

Grâce à ce système limiteur de couple 20, on peut garantir un serrage efficace et reproductible, c'est-à-dire identique, quel que soit l'utilisateur opérant le serrage, étant donné que le serrage s'opère uniquement jusqu'au déclenchement de la fonction de "débrayage" qui s'opère automatiquement dès qu'un seuil de serrage maximal ou 'optimal' est atteint, c'est-à-dire ni trop serré, ni lâche ou insuffisamment serré. Le seuil de serrage est préférentiellement préréglé, par exemple en usine, et non modifiable par l'utilisateur afin de limiter les risques de mauvais réglage par l'utilisateur susceptibles de conduisant à des serrages trop forts ou insuffisants.

D'une façon générale, le dispositif de fixation 10 selon l'invention est particulièrement bien adapté à une utilisation sur tout appareil de fourniture de gaz, que ce soit de NO gazeux, en particulier de mélange gazeux NO/N₂, ou un autre gaz, tel un ventilateur médical fournissant de l'air, de l'oxygène ou un mélange air/O₂, voire un autre gaz médical,

Lorsque l'appareil est un appareil de fourniture de NO, il peut être destiné à être raccordé au circuit patient d'un ventilateur médical fournissant un gaz respiratoire, en particulier un mélange N₂/O₂ ou de l'air, de manière à obtenir un ou des mélanges gazeux thérapeutique à base de NO, O₂ et N₂, voire aussi d'autres composés.

Le dispositif de fixation 10 selon l'invention permet de maintenir fermement l'appareil de fourniture de gaz, tel de NO ou un autre gaz, accroché à un support S allongé (i.e. longiforme ou analogue), tel un barreau de lit, de brancard ou analogue, ou une tringle T notamment dans un véhicule de transport ou analogue, avant, pendant et après traitement d'un patient par inhalation du gaz thérapeutique, y compris pendant un déplacement du patient d'un site à un autre, par exemple au sein d'un bâtiment hospitalier ou au sein d'un véhicule de transport, telle une ambulance, un avion, un hélicoptère ou autres.

## Revendications

1. Appareil médical (1) de fourniture de gaz comprenant une carcasse (2) comprenant une paroi externe (3) et un dispositif de fixation (10) à un support allongé (S), agencé sur la paroi externe (3) de ladite carcasse (2), dans lequel le dispositif de fixation (10) comprend :
- un corps de pince (12) et un élément de pince (13) mobile en pivotement (XX) l'un par rapport à l'autre,
- au moins une partie du corps de pince (12) est configurée en une première mâchoire (15) et au moins une partie de l'élément de pince (13) mobile étant configurée en une seconde mâchoire (16), lesdites première et seconde mâchoires (15, 16) étant agencées face à face pour former une pince de serrage, et
- un organe de manipulation rotatif (11), actionnable en rotation par un utilisateur, coopérant avec l'élément de pince (13), lorsqu'il est actionné, pour déplacer l'élément de pince (13) respectivement au corps de pince (12) et rapprocher ou éloigner la première mâchoire (15) de la seconde mâchoire (16),
et dans lequel :
- l'organe de manipulation rotatif (11) comprend une tête (11.1) destinée à être saisie manuellement par l'utilisateur et une tige d'actionnement (11.2) comprenant une portion filetée (11.4), et
- une pièce d'actionnement taraudée coopère avec la portion filetée (11.4) de la tige d'actionnement (11.2) et avec l'élément de pince (13) pour opérer le pivotement (XX) de l'élément de pince (13) par rapport au corps de pince (12) de manière à rapprocher ou éloigner lesdites première et seconde mâchoires (15, 16) l'une de l'autre,
**caractérisé en ce que** l'organe rotatif (11) comprend en outre un système limiteur de couple (20) agencé dans la tête (11.1) de l'organe de manipulation rotatif (11), ledit système limiteur de couple (20) comprenant un empilement de rondelles-ressorts (21) coopérant avec une rondelle de pression (24).

2. Appareil selon la revendication 1, **caractérisé en ce que** le corps de pince (12) et l'élément de pince (13) sont portés par une platine-support (14), ladite platine-support (14) étant fixée à la paroi externe (3) de ladite carcasse (2).

3. Appareil selon la revendication 1, **caractérisé en ce que** la portion filetée (11.4) de la tige d'actionnement (11.2) de l'organe de manipulation rotatif (11) est configurée pour se déplacer dans le taraudage de la pièce d'actionnement taraudée lorsque l'utilisateur applique un mouvement de vissage horaire ou antihoraire à la tête (11.1) de l'organe de manipulation rotatif (11).

4. Appareil selon l'une des revendications 1 ou 3, **caractérisé en ce que** la pièce d'actionnement taraudée est configurée être déplacée en translation, lorsque la tige d'actionnement (11.2) de l'organe de manipulation rotatif (11) vient agir sur la pièce d'actionnement taraudée.

5. Appareil selon l'une des revendications 1, 3 ou 4, **caractérisé en ce que** la pièce d'actionnement taraudée est configurée pour engendrer un déplacement angulaire (XX) de l'élément de pince (13) par rapport au corps de pince (12), et un déplacement en rapprochement ou en éloignement de la seconde mâchoire (16) par rapport à la première mâchoire (15), selon le sens d'actionnement de la tige d'actionnement (11.2) par l'utilisateur, lorsque la tige d'actionnement (11.2) de l'organe de manipulation rotatif (11) vient agir sur la pièce d'actionnement taraudée.

6. Appareil selon la revendication 1, **caractérisé en ce que** la première zone de serrage (15.1) de la première mâchoire (15) présente un profil semi-circulaire et la seconde zone de serrage (16.1) de la seconde mâchoire (16) présente un profil linéaire, ou inversement.

7. Appareil selon la revendication 1, **caractérisé en ce que** le système limiteur de couple (20) comprend un empilement de moins de 12 rondelles-ressorts, de préférence de 2 à 8 rondelles-ressorts.

8. Appareil selon la revendication 1, **caractérisé en ce que** l'empilement de rondelles-ressorts (21) vient appuyer sur la rondelle de pression (24) agencée dans la tête de l'organe (11.1) de manipulation rotatif (11).

9. Appareil selon les revendications 1 et 8, **caractérisé en ce que** la tête (11.1) de l'organe de manipulation rotatif (11) comprend un épaulement annulaire (26) interne agencé entre la rondelle de pression (24) et un élément de socle (22) solidaire de la tige d'actionnement (11.2).

10. Appareil selon la revendication 9, **caractérisé en ce que** l'épaulement annulaire (26) est traversé par au moins un logement à goupille (30) comprenant une pièce-mobile (31) mobile en translation au sein dudit logement à goupille (30), de préférence plusieurs logements à goupille (30) comprenant chacun une pièce-mobile (31).

11. Appareil selon la revendication 9, **caractérisé en ce que** l'élément de socle (22) comprend au moins un logement borgne (32) aménagé sur une surface annulaire supérieure (33) de l'élément de socle (22) située en regard de l'épaulement (26).

12. Appareil selon les revendications 10 et 11, **caractérisé en ce que** la pièce-mobile (31) est partiellement insérée dans ledit au moins un logement borgne (32) de l'élément de socle (22) lorsque le système limiteur de couple (20) est dans au moins une position embrayée, dans laquelle la tête (11.1) de l'organe de manipulation rotatif (11) est couplée à l'élément de socle (22) via l'épaulement annulaire (26), de sorte qu'un mouvement de rotation appliqué par l'utilisateur à la tête (11.1) de l'organe de manipulation rotatif (11) entraîne simultanément une rotation de la tête (11.1) de l'organe de manipulation rotatif (11) et de l'élément de socle (22).

13. Appareil selon les revendications 10 et 11, **caractérisé en ce que** la pièce-mobile (31) est positionnée en dehors dudit au moins un logement-borgne (32) de l'élément de socle (22) lorsque le système limiteur de couple (20) est dans au moins une position débrayée, dans laquelle la tête (11.1) de l'organe de manipulation rotatif (11) est découplée de l'élément de socle (22), de sorte qu'un mouvement de rotation appliqué par l'utilisateur à la tête (11.1) de l'organe de manipulation rotatif (11) n'entraîne pas l'élément de socle (22).

14. Appareil selon la revendication 13, **caractérisé en ce qu'**en position débrayée, ladite au moins une pièce-mobile (31) repousse la rondelle de pression (24) en direction de l'empilement de rondelles-ressorts (21) de sorte d'éloigner la rondelle de pression (24) de l'épaulement annulaire (26) de la tête (11.1) de l'organe de manipulation rotatif (11).

15. Appareil selon la revendication 13, **caractérisé en ce qu'**un moyen de maintien (23) permet se solidariser l'empilement de rondelles-ressorts (21) à la tige d'actionnement (11.2), de préférence l'empilement de rondelles-ressorts (21) est maintenu entre une tête (23.1) du moyen de maintien (23) et la rondelle de pression (24) agencée dans la tête de l'organe (11.1) de manipulation rotatif (11).

## Patentansprüche

1. Medizinisches Gasversorgungsgerät (1), umfassend ein Gehäuse (2), das eine Außenwand (3) und eine Befestigungsvorrichtung (10) an einem länglichen Träger (S) umfasst, die an der Außenwand (3) des Gehäuses (2) angeordnet ist, wobei die Befestigungsvorrichtung (10) umfasst:
- einen Zangenkörper (12) und ein Zangenelement (13), das in einer Schwenkbewegung (XX) zueinander beweglich ist,
- wobei mindestens ein Teil des Zangenkörpers (12) als erste Backe (15) und mindestens ein Teil des beweglichen Zangenelements (13) als zweite Backe (16) ausgebildet ist, wobei die erste und die zweite Backe (15, 16) einander gegenüberliegend angeordnet sind, um eine Klemmzange zu bilden, und
- ein drehbares Betätigungsorgan (11), das durch einen Benutzer in Drehung versetzbar ist und mit dem Zangenelement (13) zusammenwirkt, wenn es betätigt wird, um das Zangenelement (13) jeweils zum Zangenkörper (12) zu bewegen und die erste Backe (15) an die zweite Backe (16) anzunähern oder davon zu entfernen,
und wobei:
- das drehbare Betätigungsorgan (11) einen Kopf (11.1), der dazu bestimmt ist, vom Benutzer manuell ergriffen zu werden, und eine Betätigungsstange (11.2) mit einem Gewindeabschnitt (11.4) umfasst, und
- ein Betätigungsteil mit Innengewinde mit dem Gewindeabschnitt (11.4) der Betätigungsstange (11.2) und mit dem Zangenelement (13) zusammenwirkt, um das Schwenken (XX) des Zangenelements (13) in Bezug auf den Zangenkörper (12) zu bewirken, um die erste und zweite Backe (15, 16) einander anzunähern oder voneinander zu entfernen,
**dadurch gekennzeichnet, dass** das drehbare Organ (11) ferner ein Drehmomentbegrenzungssystem (20) umfasst, das im Kopf (11.1) des drehbaren Betätigungsorgans (11) angeordnet ist, wobei das Drehmomentbegrenzungssystem (20) einen Stapel Tellerfedern (21) umfasst, die mit einer Druckscheibe (24) zusammenwirken.

2. Gerät nach Anspruch 1, **dadurch gekennzeichnet, dass** der Zangenkörper (12) und das Zangenelement (13) von einer Trägerplatte (14) getragen werden, wobei die Trägerplatte (14) an der Außenwand (3) des Gehäuses (2) befestigt ist.

3. Gerät nach Anspruch 1, **dadurch gekennzeichnet, dass** der Gewindeabschnitt (11.4) der Betätigungsstange (11.2) des drehbaren Betätigungsorgans (11) so konfiguriert ist, dass er sich im Gewinde des Betätigungsteils mit Innengewinde bewegt, wenn der Benutzer eine Schraubbewegung im Uhrzeigersinn oder gegen den Uhrzeigersinn auf den Kopf (11.1) des drehbaren Betätigungsorgans (11) ausübt.

4. Gerät nach einem der Ansprüche 1 oder 3, **dadurch gekennzeichnet, dass** das Betätigungsteil mit Innengewinde so konfiguriert ist, dass es translatorisch bewegt wird, wenn die Betätigungsstange (11.2) des drehbaren Betätigungsorgans (11) auf das Betätigungsteil mit Innengewinde wirkt.

5. Gerät nach einem der Ansprüche 1, 3 oder 4, **dadurch gekennzeichnet, dass** das Betätigungsteil mit Innengewinde so konfiguriert ist, dass es eine Winkelverschiebung (XX) des Zangenelements (13) in Bezug auf den Zangenkörper (12) und eine Verschiebung in Annäherung oder Entfernung der zweiten Backe (16) in Bezug auf die erste Backe (15) erzeugt, je nach Betätigungsrichtung der Betätigungsstange (11.2) durch den Benutzer, wenn die Betätigungsstange (11.2) des drehbaren Betätigungsorgans (11) auf das Betätigungsteil mit Innengewinde wirkt.

6. Gerät nach Anspruch 1, **dadurch gekennzeichnet, dass** der erste Klemmbereich (15.1) der ersten Backe (15) ein halbkreisförmiges Profil aufweist und der zweite Klemmbereich (16.1) der zweiten Backe (16) ein lineares Profil aufweist, oder umgekehrt.

7. Gerät nach Anspruch 1, **dadurch gekennzeichnet, dass** das Drehmomentbegrenzungssystem (20) einen Stapel von weniger als 12 Tellerfedern, vorzugsweise von 2 bis 8 Tellerfedern, umfasst.

8. Gerät nach Anspruch 1, **dadurch gekennzeichnet, dass** der Stapel von Tellerfedern (21) auf der Druckscheibe (24) aufliegt, die im Kopf des drehbaren Betätigungsorgans (11.1) angeordnet ist.

9. Gerät nach den Ansprüchen 1 und 8, **dadurch gekennzeichnet, dass** der Kopf (11.1) des drehbaren Betätigungsorgans (11) einen inneren ringförmigen Absatz (26) umfasst, der zwischen der Druckscheibe (24) und einem Sockelelement (22) angeordnet ist, das fest mit der Betätigungsstange (11.2) verbunden ist.

10. Gerät nach Anspruch 9, **dadurch gekennzeichnet, dass** der ringförmige Absatz (26) von mindestens einer Stiftaufnahme (30) durchquert wird, die ein bewegliches Teil (31) umfasst, das translatorisch innerhalb der Stiftaufnahme (30) beweglich ist, vorzugsweise mehrere Stiftaufnahmen (30), die jeweils ein bewegliches Teil (31) umfassen.

11. Gerät nach Anspruch 9, **dadurch gekennzeichnet, dass** das Sockelelement (22) mindestens eine Sacklochbohrung (32) umfasst, die auf einer oberen ringförmigen Fläche (33) des Sockelelements (22) angeordnet ist, die dem Absatz (26) gegenüberliegt.

12. Gerät nach den Ansprüchen 10 und 11, **dadurch gekennzeichnet, dass** das bewegliche Teil (31) teilweise in die mindestens eine Sacklochbohrung (32) des Sockelelements (22) eingeführt ist, wenn sich das Drehmomentbegrenzungssystem (20) in mindestens einer eingerückten Position befindet, in der der Kopf (11.1) des drehbaren Betätigungsorgans (11) über den ringförmigen Absatz (26) mit dem Sockelelement (22) gekoppelt ist, so dass eine vom Benutzer auf den Kopf (11.1) des drehbaren Betätigungsorgans (11) ausgeübte Drehbewegung gleichzeitig eine Drehung des Kopfes (11.1) des drehbaren Betätigungsorgans (11) und des Sockelelements (22) bewirkt.

13. Gerät nach den Ansprüchen 10 und 11, **dadurch gekennzeichnet, dass** das bewegliche Teil (31) außerhalb der mindestens einen Sacklochbohrung (32) des Sockelelements (22) positioniert ist, wenn sich das Drehmomentbegrenzungssystem (20) in mindestens einer ausgerückten Position befindet, in der der Kopf (11.1) des drehbaren Betätigungsorgans (11) vom Sockelelement (22) entkoppelt ist, so dass eine vom Benutzer auf den Kopf (11.1) des drehbaren Betätigungsorgans (11) ausgeübte Drehbewegung das Sockelelement (22) nicht antreibt.

14. Gerät nach Anspruch 13, **dadurch gekennzeichnet, dass** in der ausgerückten Position das mindestens eine bewegliche Teil (31) die Druckscheibe (24) in Richtung des Stapels von Tellerfedern (21) zurückdrückt, um die Druckscheibe (24) vom ringförmigen Absatz (26) des Kopfes (11.1) des drehbaren Betätigungsorgans (11) zu entfernen.

15. Gerät nach Anspruch 13, **dadurch gekennzeichnet, dass** ein Haltemittel (23) es ermöglicht, den Stapel von Tellerfedern (21) an der Betätigungsstange (11.2) zu befestigen, vorzugsweise wird der Stapel von Tellerfedern (21) zwischen einem Kopf (23.1) des Haltemittels (23) und der Druckscheibe (24) gehalten, die im Kopf des drehbaren Betätigungsorgans (11.1) angeordnet ist.

## Claims

1. A medical gas supply apparatus (1) comprising a casing (2) comprising an external wall (3) and a fixing device (10) to an elongated support (S), arranged on the external wall (3) of said casing (2), wherein the fixing device (10) comprises:
- a clamp body (12) and a clamp element (13) movable in pivoting (XX) with respect to each other,
- at least one part of the clamp body (12) is configured as a first jaw (15) and at least one part of the movable clamp element (13) is configured as a second jaw (16), said first and second jaws (15, 16) being arranged face to face to form a clamping clamp, and
- a rotary manipulation member (11), actuable in rotation by a user, cooperating with the clamp element (13), when it is actuated, to move the clamp element (13) respectively to the clamp body (12) and to bring the first jaw (15) closer to or further away from the second jaw (16),
and wherein:
- the rotary manipulation member (11) comprises a head (11.1) intended to be grasped manually by the user and an actuating rod (11.2) comprising a threaded portion (11.4), and
- a tapped actuating part cooperates with the threaded portion (11.4) of the actuating rod (11.2) and with the clamp element (13) to operate the pivoting (XX) of the clamp element (13) with respect to the clamp body (12) so as to bring said first and second jaws (15, 16) closer to or further away from each other,
**characterized in that** the rotary member (11) further comprises a torque limiting system (20) arranged in the head (11.1) of the rotary manipulation member (11), said torque limiting system (20) comprising a stack of disc springs (21) cooperating with a pressure washer (24).

2. The apparatus according to claim 1, **characterized in that** the clamp body (12) and the clamp element (13) are carried by a support plate (14), said support plate (14) being fixed to the external wall (3) of said casing (2).

3. The apparatus according to claim 1, **characterized in that** the threaded portion (11.4) of the actuating rod (11.2) of the rotary manipulation member (11) is configured to move in the tapping of the tapped actuating part when the user applies a clockwise or counter-clockwise screwing movement to the head (11.1) of the rotary manipulation member (11).

4. The apparatus according to one of claims 1 or 3, **characterized in that** the tapped actuating part is configured to be moved in translation when the actuating rod (11.2) of the rotary manipulation member (11) acts on the tapped actuating part.

5. The apparatus according to one of claims 1, 3 or 4, **characterized in that** the tapped actuating part is configured to generate an angular displacement (XX) of the clamp element (13) with respect to the clamp body (12), and a displacement in approach or in removal of the second jaw (16) with respect to the first jaw (15), according to the direction of actuation of the actuating rod (11.2) by the user, when the actuating rod (11.2) of the rotary manipulation member (11) acts on the tapped actuating part.

6. The apparatus according to claim 1, **characterized in that** the first clamping zone (15.1) of the first jaw (15) has a semi-circular profile and the second clamping zone (16.1) of the second jaw (16) has a linear profile, or vice versa.

7. The apparatus according to claim 1, **characterized in that** the torque limiting system (20) comprises a stack of less than 12 disc springs, preferably from 2 to 8 disc springs.

8. The apparatus according to claim 1, **characterized in that** the stack of disc springs (21) bears on the pressure washer (24) arranged in the head of the rotary manipulation member (11.1).

9. The apparatus according to claims 1 and 8, **characterized in that** the head (11.1) of the rotary manipulation member (11) comprises an internal annular shoulder (26) arranged between the pressure washer (24) and a base element (22) integral with the actuating rod (11.2).

10. The apparatus according to claim 9, **characterized in that** the annular shoulder (26) is traversed by at least one pin housing (30) comprising a movable part (31) movable in translation within said pin housing (30), preferably several pin housings (30) each comprising a movable part (31).

11. The apparatus according to claim 9, **characterized in that** the base element (22) comprises at least one blind bore (32) arranged on an upper annular surface (33) of the base element (22) located opposite the shoulder (26).

12. The apparatus according to claims 10 and 11, **characterized in that** the movable part (31) is partially inserted into said at least one blind bore (32) of the base element (22) when the torque limiting system (20) is in at least one engaged position, in which the head (11.1) of the rotary manipulation member (11) is coupled to the base element (22) via the annular shoulder (26), so that a rotational movement applied by the user to the head (11.1) of the rotary manipulation member (11) simultaneously causes a rotation of the head (11.1) of the rotary manipulation member (11) and of the base element (22).

13. The apparatus according to claims 10 and 11, **characterized in that** the movable part (31) is positioned outside said at least one blind bore (32) of the base element (22) when the torque limiting system (20) is in at least one disengaged position, in which the head (11.1) of the rotary manipulation member (11) is uncoupled from the base element (22), so that a rotational movement applied by the user to the head (11.1) of the rotary manipulation member (11) does not drive the base element (22).

14. The apparatus according to claim 13, **characterized in that** in the disengaged position, said at least one movable part (31) pushes back the pressure washer (24) in the direction of the stack of disc springs (21) so as to move the pressure washer (24) away from the annular shoulder (26) of the head (11.1) of the rotary manipulation member (11).

15. The apparatus according to claim 13, **characterized in that** a retaining means (23) allows the stack of disc springs (21) to be secured to the actuating rod (11.2), preferably the stack of disc springs (21) is maintained between a head (23.1) of the retaining means (23) and the pressure washer (24) arranged in the head of the rotary manipulation member (11.1).
